# EUROPEAN PATENT APPLICATION

(11) **EP 4 011 295 A1**
(43) Date of publication of application: **15.06.2022**
(21) Application number: 21211893.9
(22) Date of filing: 02.12.2021
(51) Int. Cl.: A61B 6/14, A61B 6/00, A61B 6/03

(54) **METHOD AND APPARATUS FOR DIGITAL PANORAMIC IMAGING WITH VARIABLE THICKNESS OF THE PANORAMIC LAYER AND COMPUTER PROGRAM PRODUCT**

(30) Priority: 09.12.2020 IT 202000030221
(71) Applicant: De Gotzen S.r.l., 21057 Olgiate Olona (Varese) (IT)
(72) Inventor: GIANI, Claudio, 21052 Busto Arsizio (Varese) (IT); ROTONDO, Giuseppe, 20090 Settala (Milano) (IT); VENTURINO, Gianfranco, 20159 Milano (MI) (IT)
(74) Representative: Herrmann, Franz

(57) **Abstract**

A method and an apparatus for digital panoramic imaging in the head region of a patient is proposed. The apparatus comprises an X-ray source and X-ray sensor (12) as well as a control unit for processing the frames (14) provided by the sensor (12), in particular for performing a shift-and-add-operation on the frames (14) for generating a digital panoramic image of a focal plane (34) situated in a dental arch of the patient (2). In the digital shift-and-add operation, the frames (14) are weighted by weighting coefficients obtained from a weighting function (42) having, along a line extending across the frame (14)in the moving direction of the sensor (12), a maximum in a central region of the frame (14) and smaller values towards the edge of the frame (14), thus reducing the effective width of the sensor (12). The modulation of the effective width of the sensor (12) can be used for varying the width of the sharply imaged panoramic layer.

## Description

Method for generating a digital panoramic image of the head region of a patient based on data obtained from an apparatus comprising:
- a source, which generates X-ray radiation;
- a sensor provided with a two-dimensional array of pixels, which are suitable for generating frames based on the X-ray radiation, which is generated by the source and passes through the patient;
- a supporting structure for arranging the source and the sensor thereon in such a way as to be opposed to each other and for moving the source and the sensor around the head of the patient;
the method comprising the use of a processing unit for performing a shift-and-add-operation on the frames for generating the digital panoramic image of a focal plane in the head region of the patient.

The invention further relates to an apparatus for performing such a method and to a corresponding computer program product.

Such an apparatus is known from US 8,693,624 B2. The known apparatus is used for generating panoramic images of the dentition of a patient. A problem of the known apparatus is, that the thickness of the sharply imaged layer may vary.

US 4,985,907 discloses that the thickness of the sharply imaged layer is directly proportional to the distance of the instantaneous center of rotation from the object plane and inversely proportional to the magnification and to the width of the ray beam.

US 6,744,847 B2 discloses an apparatus and a method for panoramic dental imaging comprising a shutter for shaping the X-ray beam. The shutter narrows the X-ray beam in the front area of the dental arch in order to increase the thickness of the sharply imaged layer compared to the two sides of the dental arch. The operation of the shutter is synchronized with the movement of the rotation by a complex mechanism, that implies a predetermined spatial relationship between patient and apparatus.

Proceeding from this related art, the present invention seeks to provide a method and an apparatus that can be more easily adapted to the specific needs of an individual panoramic imaging operation. The present invention further seeks to provide a corresponding computer program product.

This object is achieved by a method, an apparatus and a computer program product having the features of the independent claim. Advantageous embodiments and refinements are specified in claims dependent thereon.

In the method, the digital shift-and-add operation is performed such that the digital images data of frames are weighted by weighting coefficients obtained from a weighting function having, along a line extending across the frame in the moving direction of the sensor, a maximum in a central region of the frame and smaller values towards the edge of the frame. The pixels situated off-center are thus contributing less to the reconstruction of the panoramic image, which modulates the effective sensor width in the moving direction of the sensor. By varying the width of the weighting function, the thickness of the panoramic layer can thus be adapted to the specific needs of an individual panoramic imaging operation.

The weighting function may particularly comprise a maximum along a central vertical line of the frames and may decrease to smaller values towards the vertical boundaries of the frames.

The weighting function can also be a two-dimensional weighting function that varies in the horizontal and vertical direction. Thus, the thickness of the panoramic layer can vary in the vertical direction which can be useful if the thickness of the panoramic layer may be different, for instance, for the lower and upper jaw.

The weighting functions are generally symmetric with regard to the maximum. That means that the slopes of the weighting functions, which are decreasing from a central maximum towards vertical boundaries along a horizontal line, are symmetric with respect to the maximum. In some cases, however, the weighting function may also comprise an asymmetric shape. The slopes of the asymmetric weighting function, which are decreasing from a central maximum towards the vertical boundaries along a horizontal line, are then asymmetric with respect to the maximum of the asymmetric weighting function. This can be particularly useful in transition regions, where the pixels on both sides of the maximum need to be treated differently.

In most cases, the weighting functions associated with frames that are taken at positions, which are symmetric with respect to a median plane of the patient, are the same or mirrored with respect to the median plane. Such an arrangement takes into account the usual symmetry of the dental arch.

In one particular embodiment of the method, the weighting function is exclusively applied to frames taken in a region around the median plane of the patient. In the central region of the dental arch located around the incisors the sequence of teeth in the upper jaw is frequently offset from the sequence of teeth in the lower jaw. By enlarging the thickness of the panoramic layer in the central region of the dental arch, both the lower and upper jaw can be imaged by one single scan.

The weighting function is particularly applied to frames, in which the angle between the normal of the sensor in the associated position and the median plane is in a range extending from 0° to an upper limit lying between 30° to 40°.

Accordingly, in some cases, the full-width-half-maximum (FWHM) of the weighting function is at its minimum for the frame taken at the position where the scan trajectory of the sensor intersects the median plane of the patient.

The FWHM of subsequent weighting functions may decrease gradually from one weighting function to the next weighting function towards the minimum ensuring a smoothly increasing width of the panoramic layer. Alternatively, the FWHM may decrease in a single step or in multiple steps to the minimum, wherein the FWHM remains constant between the steps.

In one embodiment, the frame columns are weighted by multiplying pixel values of the frame columns with a single weighting coefficient provided by the weighting function for the respective frame column.

The shift-and-add operation generally comprises the following operations:
- aligning the frame with the panoramic image;
- weighting the pixel values of the frame; and
- adding the weighted pixel values to the panoramic pixels of the panoramic image.

The frame is particularly aligned by shifting the frame to a determined or predetermined position on the panoramic image, and the pixel values of the frame are added to panoramic pixel values of corresponding panoramic pixels of the panoramic image. In case of a pixel mismatch between pixels of the frame and panoramic pixels, however, the alignment and adding is performed by distributing the pixel values of a frame column to the columns of the panoramic image, which are overlapped by the frame column, wherein the splitting is performed by splitting the values of the frame column proportional to the offset in the moving direction of the sensor. In both cases, the pixel values of the frame are weighted before or after aligning the frame.

The frame can also be aligned by interpolating the pixel values of the frame along a generally horizontal frame line and by using the interpolation function to resample the pixel values of the frame to the panoramic pixels. In this case, the original pixel values of the frame before interpolation or the resampled pixel values of the frame after interpolation are weighted.

For an appropriate sampling of the dental arch, in particular for avoiding oversampling the dental arch, the frames are obtained such that the movement between two consecutive frames is not less than half the pixel width and stays less than the full width of the pixel, wherein the pixel width is the dimension of the pixels in the moving direction of the sensor.

The particular processing of the image data can be used for imaging any region in a patient's head, but is generally used for imaging a patient's dentition.

An apparatus for performing the method can be an apparatus for digital panoramic imaging in the head region of a patient comprising:
- a source, which generates X-ray radiation;
- a sensor provided with a two-dimensional array of pixels, which are suitable for generating frames based on the X-ray radiation, which is generated by the source and passes through the patient;
- a support structure for arranging the source and the sensor thereon in such a way as to be opposed to each other and for moving the source and the sensor around the head of the patient; and
- a processing unit arranged for performing a shift-and-add-operation on the frames for generating a digital panoramic image of a focal plane in the head region of the patient.

In such an apparatus the processing unit can perform the method for generating the panoramic images.

In one embodiment, the supporting structure of the apparatus comprises:
- a rotary unit for arranging the source and the sensor thereon in such a way as to be opposed to each other; and
- a support for supporting the rotary unit, wherein a motor driven translation unit and a motor driven rotation unit are interposed between the rotary unit and the supporting structure for moving the source and the sensor around the head of the patient.

Such a supporting structure allows to perform a roto-translational movement of the source and the sensor around the head of the patient.

The apparatus may further comprise a control unit, which controls the source, the sensor, the translation unit and the rotation unit and which is provided with the processing unit, thus integrating these functionalities in one device.

Further advantages and properties of the present invention are disclosed in the following description, in which exemplary embodiments of the present invention are explained in detail based on the drawings:
- Figure 1: shows an apparatus for panoramic imaging;
- Figure 2: illustrates the movement of X-ray source and sensor and the trajectory of the mechanical rotation axis during scanning;
- Figure 3: illustrates the effect of the effective width of the sensor on the thickness of the sharply imaged layer;
- Figure 4: shows the usual variation of the thickness of the sharply imaged layer along a dental arch of a patient without compensation;
- Figure 5: is a flow diagram illustrating the method steps taken during the construction of the panoramic image;
- Figure 6: illustrates the generation of the panoramic image based on individual frames using a weighting function, which reduces the effective width of the sensor;
- Figure 7: illustrates the alignment of the frames to the panoramic image according a first method;
- Figure 8: illustrates the alignment of the frames to the panoramic image according to an alternative method;
- Figure 9: illustrates the variation of the weighting function used during scanning along the dental arch; and
- Figure 10 to 12: shows alternative shapes of the weighting functions.

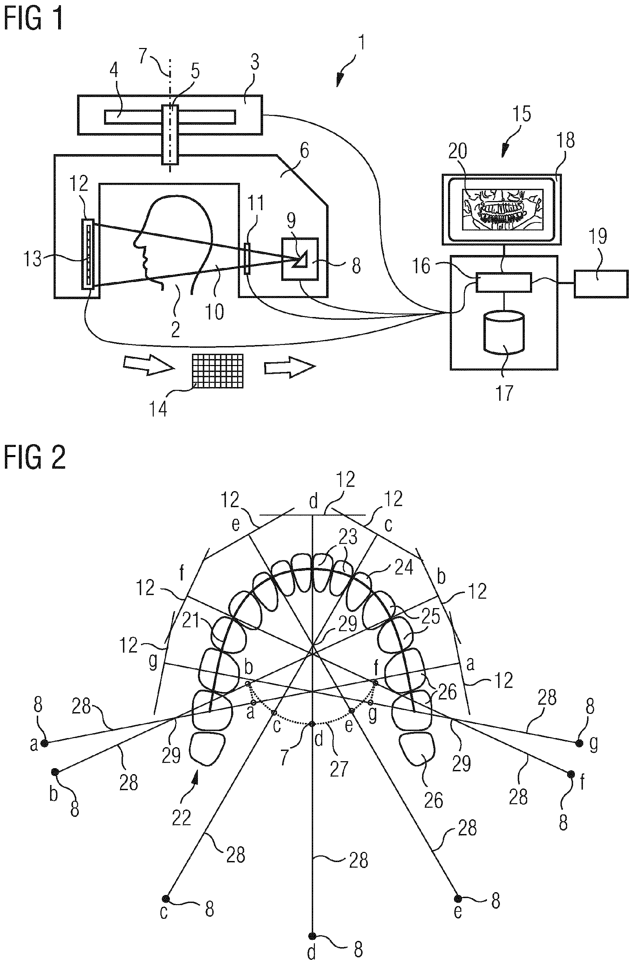

Figure 1 shows an apparatus 1 for digital panoramic imaging in the head region of a patient 2. The apparatus 1 comprises a support 3. The support 3 is equipped with a linear drive unit 4. The drive unit 4 allows to move a rotational drive unit 5 in a lateral x- and y-directions, whereas the rotational unit 5 moves a rotary arm 6 around a rotational axis 7, which is oriented in a z-direction. At one end of the rotary arm 6, a source 8 for generating X-rays is situated. The source 8, for instance an X-ray tube, comprises a focal spot 9, from which an X-ray beam 10 emerges. The X-ray beam 10 passes through a collimator 11 which provides the beam 10 with a fan or cone shape. The beam 10 then passes through the head of the patient 2 and hits a sensor 12 for detecting X-rays. The sensor 12 may be a two-dimensional sensor comprising an array of pixels 13 that are usually arranged in lines and columns. The sensor 12 has a width that extends in a horizontal moving direction over several columns and a height that extends in vertical direction over several lines. The sensor 12 can be a sensor directly converting X-rays into electrical signals within the pixels 13 or a sensor comprising a scintillator for converting X-rays into light detectable by the pixels 13 of the sensor 12. The pixels 13 can be used for producing electrical signals that correlate with the quantity of X-ray radiation incident on the pixels 13 during a given measurement period. The electrical signal can be converted into digital pixel values. A two-dimensional representation of pixel values taken within the same measurement period is called a frame 14. The frames 14 are read out from the sensor 12 by a control unit 15. The control unit 15 can be a computer comprising a processing unit 16 and a data storage 17 and may comprise a display 18 for displaying a graphical user interface. The control unit 15 may further comprise input means 19 for inputting data to the control unit 15. The frames 14 can be stored in the data storage 17 and are processed to generate a panoramic image 20, for instance, of the dentition of the patient 2. The panoramic image 20 can be shown to the operator on the display 18. The control unit 15 can also be used for controlling the movement of the linear drive unit 4, the rotation performed by the rotational drive unit 5, the X-ray emission of the source 8 and the operational parameters of the collimator 11 determining the shape and the dimension of the beam 10.

Figure 2 illustrates the movement of the sensor 12 and source 8 during a scanning operation for generating the panoramic image 20 of a panoramic layer 21 within a dental arch 22 of the patient 2. The complete dental arch 22 of an adult patient 2 along one jaw consists of two incisors 23, one canine 24, two premolars 25 and three molars 26 in each half of the dental arch 22. The third molar 26 is also called the wisdom tooth and is not always present. During the scanning operation the rotary arm 6 performs a roto-translational motion, in which the rotary arm 6 is pivoted around the rotational axis 7 and in which the rotational axis 7 is translated in a plane perpendicular to the rotational axis 7. In moving the rotational axis 7 along a trajectory 27, various requirements are fulfilled: A central ray 28 of the beam 10 is essentially kept at a right angle with the panoramic layer 21 to be imaged. Furthermore the distance between the panoramic layer 21 and the sensor 12 along the central ray 28 can be kept roughly constant and thus also the magnification ratio that is defined as the ratio SD/SO, wherein SD is the distance between the source 8, in particular the focal spot 9, and the sensor 12 and wherein SO is roughly the distance between the source, in particular the focal spot 9, and the panoramic layer 21 to be imaged.

It should be noted that the central rays 28 at the positions c to e intersect in a common virtual rotation center 29 that is spaced apart from the mechanical rotation center formed by the rotational axis 7. This virtual rotation center 29 need not always to be located between mechanical rotation center and sensor 12, but can also move to a position between source 8 and mechanical rotation center. If the mechanical rotation center formed by rotational axis 7 moves from position a to position b or from position f to g along the trajectory 27, the virtual rotation center 29 moves to a position between source 8 and mechanical rotation center.

The pivotation angle for the pivotation around the virtual rotation center 29 is thus larger in the region of the incisors 23, canines 24 and premolars 25 than the pivotation angle in the region of the molars 26.

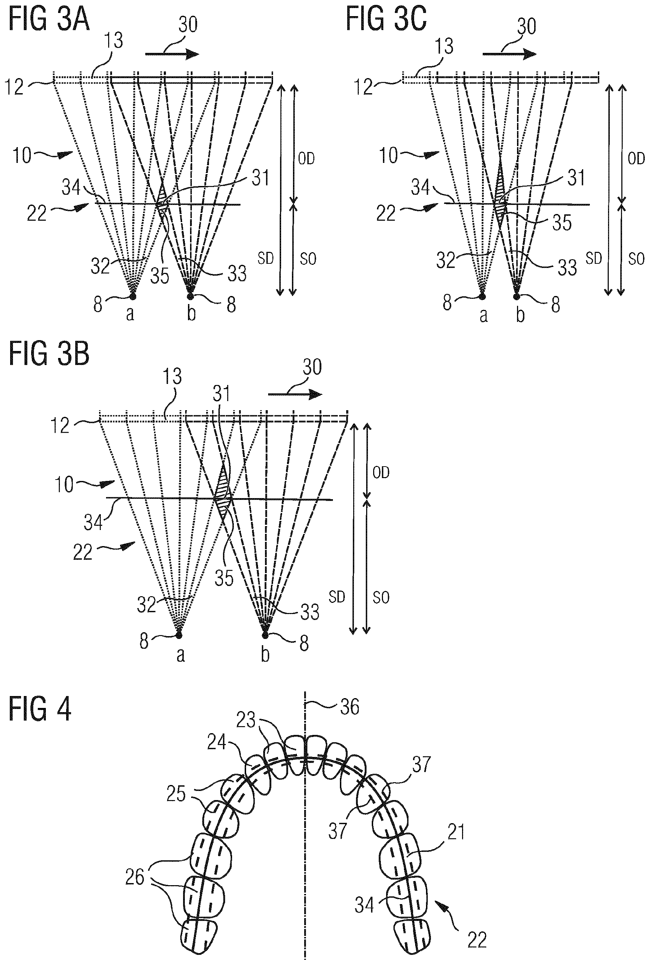

Regarding the panoramic layer 21, it has also to be taken into account that the panoramic layer 21 comprises an extension in the axial direction of the beam 10 resulting in some thickness of the panoramic layer 21. Figure 3 illustrates these facts. For the sake of clarity, the components and their spatial relation are not depicted on scale but shown in a manner intended to make the situation clear.

Figure 3A shows a source 8 and a sensor 12 moving along a dental arch 22 in a moving direction 30 from a position "a" to a position "b". The beam 10 and sensor 12 at position "a" are drawn in dotted lines whereas the beam 10 and sensor 12 at position "b" are drawn in dashed lines. For the sake of simplicity, the dental arch 22 is depicted by a straight solid line. This line also indicates the image plane of the panoramic image 20. The panoramic image 20 is composed of panoramic pixels 31 that can be thought to be located in the dental arch 22.

For constructing the panoramic image 20, the pixel values of the panoramic pixels 31 are constructed by adding the pixel values of pixels 13 which are associated with partial beams 32 and 33. Partial beams 32 and 33 intersect at the panoramic pixel 31 and impinge on the sensor 12 at position "a" and position "b" at different pixels 13 of the sensor 12.

If the pixel values of the respective pixels 13 at position "a" and position "b" are added to the pixel value of the panoramic pixel 31 of the panoramic image 20, the imaging of the X-ray absorption performed by the layer of material at the position of the panoramic pixel 31 is enhanced whereas the imaging of the absorption by material further away is blurred. The pixels 31 therefore define a focal plane 34, which extends through the dental arch 22 and defines the region where the imaging is sharpest. In consequence, the material in the focal plane 34, in which the partial beams 32 and 33 intersect, are imaged most sharply whereas the sharpness of the imaging decreases in an axial direction at right angle to the focal plane 34. The extension of the sharply imaged region in axial direction is indicated by the diamond shaped hatched intersection region 35 of the partial beams 32 and 33 .

It should be noted that the distance OD between the focal plane 34 and the sensor 12, cannot always be kept constant since the individual dental arches 22 of individual patients 2 may vary from the shape of a generic template for the dental arch 22. It may therefore happen that the distance OD is smaller than shown in Figure 3A. Figure 3B illustrates such a situation. Here the distance OD between the focal plane 34 and the sensor 12 is smaller than the distance OD in Figure 3A. In Figure 3B, the extension of the sharply imaged region is indicated by the cross-hatched intersection region 35 that is formed by the intersection of the partial beams 32 and 33. By comparing Figure 3A with Figure 3B, it can be recognized that the axial extension of the intersection region 35 from Figure 3B is larger than the axial extension of the intersection region 35 from Figure 3A. The thickness of the sharply imaged panoramic layer 21 therefore increases with decreasing distance OD between sensor 12 and focal plane 34.

It should also be noted that the thickness of the sharply imaged panoramic layer 21 increases with increasing distance SO between source 8 and focal plane 34 and decreases with increasing magnification ratio M which is more specifically determined by the ratio of the distance SD between the source 8 and the sensor 12 to the distance SO between source 8 and focal plane 34 (M=SD/SO)

The reduced thickness of the panoramic layer 21 can be compensated, though, by performing a spatial selection of the pixels 13 that are contributing to the panoramic image 20. In particular, the spatial extension of the set of contributing pixels 13 in the moving direction 30 of the sensor 12 can be restricted for enlarging the thickness of the sharply imaged region. The effect is illustrated in Figures 3C. The distances between source 8, focal plane 34 and sensor 12 in Figure 3C are the same as in in Figure 3A. In contrast to Figure 3A, only the four central pixels 13 of the sensor 12 are used for generating the panoramic image 20. As can be recognized from Figure 3C, the axial extension of the intersection region 35 is increased in comparison with the intersection region 35 of Figure 3A.

Thus, the thickness of the imaged panoramic layer 21 can be increased by restricting, in the moving direction 30, the spatial extension of the set of pixels 13 that are contributing to the pixel value of the panoramic pixel 31, which has a similar effect as reducing the width of the sensor 12 in the moving direction 30.

Reducing the effective width of the sensor 12 is advantageous under various aspects. Figure 4 shows the dental arch 22 together with a median plane 36 of the patient 2. The focal plane 34 of the panoramic layer 21 is depicted by a solid line extending along the dental arch 22. The focal plane 34 is strongly curved in the region of incisors 23, canines 24 and premolars 25. Referring to Figure 2, it was explained that the source 8 and the sensor 12 perform a pivotation around a virtual rotation center 29. In the regions of the incisors 23, canines 24 and premolars 25, the pivotation angle is large compared to the pivotation angle used in the region of the molars 26. The large pivotation angle corresponds to the situation depicted in Figure 3A, where the source 8 and the sensor 12 are moved by a large distance between positions a and b, whereas the pivotation around the virtual rotation center 29 in the region of the molars 26 corresponds to the situation illustrated in Figure 3C, where the source 8 and the sensor 12 are moved by a comparatively smaller distance. In consequence, the thickness of the panoramic layer 21 decreases in the region of the incisors 23 as compared to the region of the molars 26. Furthermore, since the pixels 13 of the sensor 12 are located in a plane, the curvature of the focal plane 34 results in an increase of the distance between the off-center pixels 13 and the focal plane 22. These effects cause the panoramic layer 21 to narrow down in the region of the incisors 23 as depicted in Figure 4, where boundaries 37 of the panoramic layer 21 are depicted by two dashed lines below and above the focal plane 34. There is consequently a need to enlarge the thickness of the panoramic layer 21 in the region of the incisors 23.

A need to enlarge the thickness of the panoramic layer 21 further arises in the region of the incisors 23, since the teeth of the upper jaw and the teeth of the lower jaw are not always aligned. It might therefore become necessary to enlarge the thickness of the panoramic layer 21 in the region of the incisors 23 in order to enable a sharp imaging of upper and lower jaw in a single scan.

Therefore, a strong interest exists in enlarging the thickness of the panoramic layer 21 next to the median plane 36 of the dental arch 22.

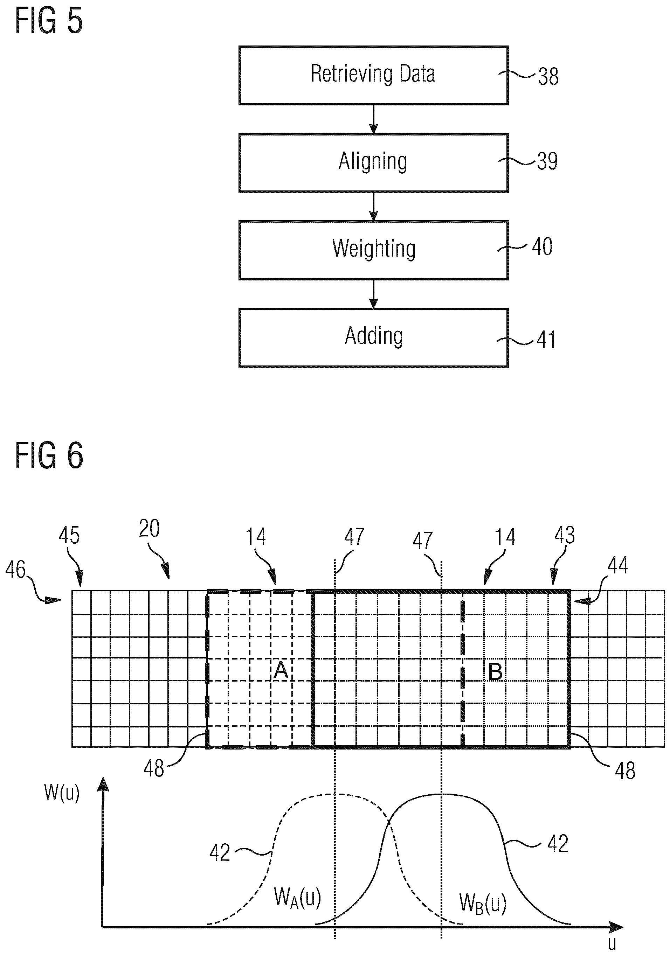

The enlargement of the panoramic layer 21 can be achieved by processing the frames 14 as depicted in Figure 5. In a first retrieval step 38 the frames 14 are retrieved from the data storage 17 or from the sensor 12. In a subsequent alignment step 39 the frames 14 are aligned with the panoramic image 20, followed by a weighting step 40, in which the pixel values are weighted, as will be explained in more detail further down. The processing of the frames 14 terminates with an adding step 41, in which the pixel values of the frames 14 are added to the pixel values of the panoramic image 20 for generating the final panoramic image 20.

In the weighting step 40, the pixel values of the frames 14 are multiplied by weighting coefficients that are determined by a weighting function 42. Figure 6 illustrates this approach. Figure 6 shows two frames 14 that are already aligned with the panoramic image 20. Theses frames 14 have frame columns 43 and frame lines 44. Figure 6 shows only two frames 14 that are designated as A and B and two associated weighting functions 42, namely W_{A}(u) and W_{B}(u), wherein u is the length of the panoramic image 20 along the trajectory of the sensor 12. The panoramic pixels 31 are arranged in panoramic columns 45 and panoramic lines 46.

The weighting functions 42 depend only on the position variable u in the moving direction 30 of the sensor 12. The weighting functions 42 generally comprise a maximum along a central vertical line 47 of the frames 14 and decreases to smaller values towards vertical boundaries 48 of the frames 14. The weighting functions 42 may, for instance, have a maximum with the value of one and may decrease to zero at the boundaries 48. The weighting functions 42 will generally be symmetric with respect to the central vertical line 47.

Figure 6 shows the frames 14 after the alignment step 39, in which the frames 14 are aligned with the panoramic image 20 in preparation of the adding step 41. In the weighting step 40, the pixel values in the frame columns 43 are weighted by weighting coefficients that are determined by the weighting functions 42 that are associated with the respective frames 14. The weighting coefficients are the values of the weighting function 42 at the position u, if the respective frame column 43 is located at position u. Thus, the pixel values of frame 14 at the position A are multiplied with the weighting coefficients of weighting function W_{A} and the pixel values of frame 14 at the position B are multiplied with the weighting coefficients of weighting function W_{B}. After weighting the pixel values of the frames 14 the pixel values of the frames 14 are added to the pixel values of the panoramic pixels 31.

These method steps are performed if the pixels 13 coincide with the panoramic pixels 31.

It may happen that an offset occurs between the panoramic pixels 31 and the pixels 13 of the sensor 12 and thus of the frame 14. For instance, the pixels 13 may be offset from the panoramic pixels 31 by a fraction of the distance between pixels 13. In this case, the alignment of the frames 14 to the panoramic image 20 may be performed in various ways.

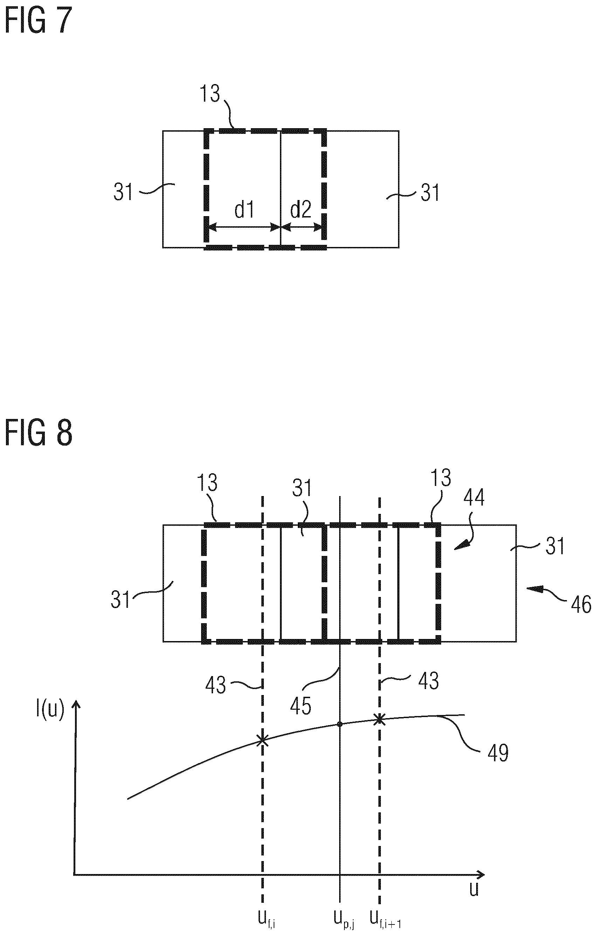

For instance, the pixel values of a particular frame column 43 may be distributed to the panoramic columns 45, which are overlapped by the frame column 43, wherein the splitting is performed by splitting the values of the frame column 43 proportional to the offset in the moving direction 30 of the sensor 12. This approach is illustrated in Figure 7, where a single pixel 13 of a frame column 43 is depicted in a dashed line. This pixel 13 is overlapping two panoramic pixels 31 depicted in solid lines. The overlap with a first pixel 31 is proportional to the distance d1 and the overlap with the second pixel 31 is proportional to the distance d2. A first portion of the pixel values of pixel 13 is then added to the first pixel 31 and a second portion of the pixel value of pixel 13 is then added to the second pixel 31. The first portion is determined by d1/(d1+d2) and the second portion is determined by d2/(d1+d2). Here, the weighting step 40 can be performed before the alignment step 39 or after the alignment step 39.

Alternatively, as shown in Figure 8, an interpolation function 49 can be fitted to the pixel values of the pixels 13 of a frame line 44. These pixels values are shown in Figure 8 to be located at positions u_{f,i}. The index i is the index of the frame column 43 and the index f shall indicate that the position is a position associated with a frame 14. The interpolation function 49 can then be used for determining the pixel values at the position u_{p,j} of the panoramic pixels 31 along the corresponding panoramic line 46, wherein j is the index of the panoramic column 45 associated with the respective panoramic pixel 31. The index p shall indicate that the position is associated with the panoramic image 20.

The weighting function 42 can be applied to the original pixel values of the frames 14 or to the interpolated pixel values at the position u_{p,j} of the panoramic pixels 31.

It should be noted that also a two-dimensional interpolation function can be determined for all pixel values of a frame 14 .

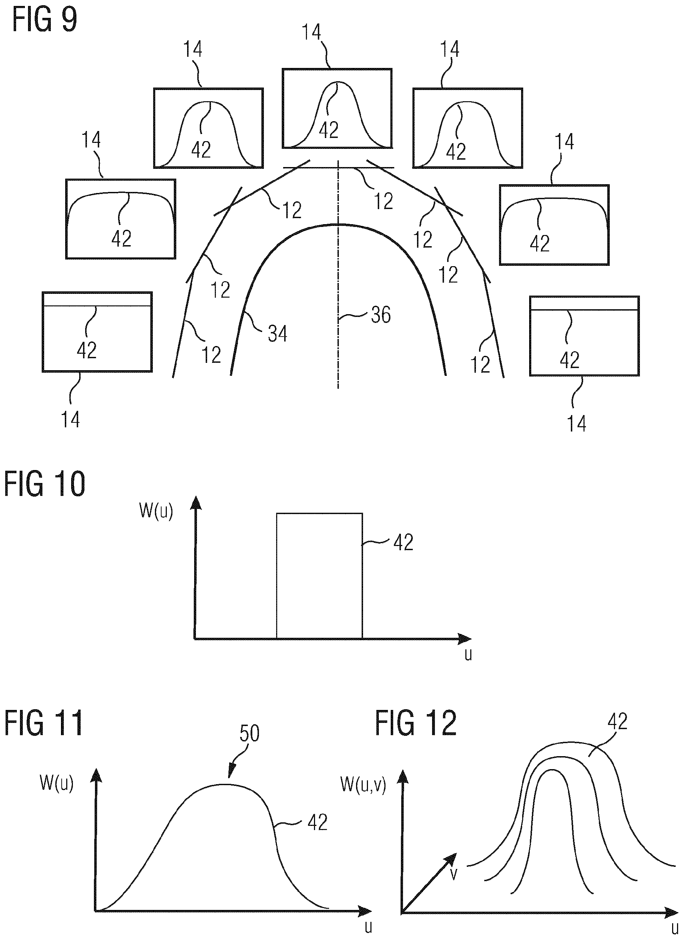

The weighting function 42 can vary along the dental arch 22. Figure 9 shows the focal plane 34 of the panoramic layer 21 together with the sensor 12 at various positions along the dental arch 22 and the associated weighting functions 42. The full-width-half-maximum (FWHM) of the weighting functions 42 increases with increasing distance from the median plane 36. The weighting functions 42 associated with frames 14 that are taken at positions, which are symmetric with respect to the median plane 36 of the patient, are generally identical or mirrored with respect to the median plane 36. That means that the same weighting functions are used on both halves of the dental arch 22, if symmetric weighting functions 42 are used, and that the weighting functions 42 are mirrored with respect to the median plane 36, if asymmetric weighting functions 42 are used.

As an alternative to a smooth slope from the maximum to a minimum at the boundaries 48, the weighting functions 42 may also comprise a rectangular shape with lateral jumps to the minimum as depicted in Figure 10.

It should further be noted that that distance between the focal plane 34 to be imaged and the pixels 13 varies over the sensor 12 due to the curvature of the focal plane 34 and due to the angular extension of the beam 10. Referring to Figure 2, it can be recognized that the distance between the pixels 13 of the sensor 12 and the panoramic layer 21 is different for the central ray 28 and off-center rays that impinge on the vertical boundaries 48 of the sensor 12. The variation of the distance causes a distortion in the panoramic image 20. Thus, it can be advantageous to reduce the contribution of the off-center pixels 13 to the panoramic image 20.

In some cases, the weighting function 42 may also comprise an asymmetric shape, as shown in Figure 11. The asymmetric weighting function 42 comprises slopes, which are decreasing along a horizontal line from a central maximum 50 to the vertical boundaries 48 and are asymmetric with respect to the maximum 50 of the asymmetric weighting function 42. Such an asymmetric shape may become useful in a transition region between a region with no weighting at all, equivalent to a weighting function 42 equal to one for all pixel values, and a region with weighting functions 42 having a rectangular shape as depicted in Figure 10. The asymmetric weighting function 42 may also be useful where the off-center pixels 13 are to be treated differently on both vertical boundaries 48 since the distance between dental arch 22 and the sensor 12 is different for both vertical boundaries 48, which is the case in the region of the canines 24, where the curvature of the dental arch 22 is greatest.

The weighting function 42 may also be a two-dimensional weighting function W(u,v) which depends not only on the position along the generally horizontal moving direction 30 but also on the position at right angle to the moving direction 30. Such a two-dimensional weighting function 42 is shown in Figure 12. By using such a two-dimensional weighting function 42 the thickness of the panoramic layer 21 may be different for upper and lower jaw. The use of such weighting functions 42 allows to enlarge the width of the panoramic layer 21 in the region of the incisors 23 in order to image the lower and upper jaw in a single scan.

It should be noted that the panoramic image 20 can be generated during the scan or directly after the scan with predetermined weighting functions 42. The operator of the apparatus 1 may, however, also have the option to adapt the used weighting functions 42 to specific needs. Thus, the operator may be able to select appropriate weighting functions 42 for widening up or narrowing down the width of the panoramic layer 21. For instance, the apparatus 1 may provide a first panoramic image 20, that is displayed on the display 18. If the panoramic image 20 meets the requirements and is accepted, the examination can be terminated. If, however, there is a need to improve the panoramic image 20, a second and further panoramic images 20 can be generated using modified weighting functions 42. The selection or modification of the weighting functions 42 can also be done by the control unit 15 based on a selected width of the panoramic layer 21, which the operator deems to be appropriate for the examination of a particular patient 2.

It should also be noted that the control unit 15 may also be able to modify the width of the beam 10 during a scan. The modification can also be used for adjusting the thickness of the panoramic layer 21 in a certain region of the dental arch 22. The modification can be performed by setting the width of the beam 10 via the collimator 11. For instance, if the width of the weighting function 42 is restricted such that the pixels 13 along the vertical boundaries 48 are not contributing to the panoramic image 20, the width of the beam 10 can be narrowed down such that the pixels 13 along the vertical boundaries are no longer exposed to beam 10. One advantage of using the weighting functions 42 is also that the effective width of the sensor 12 can be modulated faster and more precisely than the modulation of beam width via adjustment of the collimator 11, where the inertia of the mechanism moving the beam limiting blades restricts the swiftness of the beam adjustment.

The frames 14 can also be used for constructing more than one panoramic image 20 of various panoramic layers 21 which extend essentially in parallel along the dental arch 22. In such an embodiment, weighting functions adapted to each panoramic layer 21 can be used.

The control unit 15 can be a computer having at least one physical or logical processor. The control unit 15 can also be implemented on several physical computers or can be an integrated device including the display 18.

It should also be noted that the method can be implemented by a computer program product, that contains code for implementing the method described herein, if the code is executed by a processor either in the control unit 15 or in some separate data processing unit. In some embodiments, the computer program product may be code stored on a computer readable data carrier such as a disc, a compact disc or a digital versatile disc or the like. In other embodiments, the computer program product may also be code stored on a data storage unit on a server or an array of servers. Such a data storage unit may be a hard disc or an array of hard disc or the like. In further embodiments, the data carrier may also be an electrical carrier signal that can be used for transferring the code from a server, which can be used for downloading the program code to a client.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, components or groups described in conjunction with a particular aspect, embodiment or example of the invention are to be understood to be applicable to any other aspect, embodiment or example described herein unless incompatible therewith.

## Claims

1. Method for generating a digital panoramic image (20) of the head region of a patient (2) based on data obtained from an apparatus (1) comprising:
- a source (8), which generates X-ray radiation;
- a sensor (12) provided with a two-dimensional array of pixels (13), which are suitable for generating frames (14) based on the X-ray radiation, which is generated by the source (8) and passes through the patient (2);
- a supporting structure (3,4,5,6) for arranging the source (8) and the sensor (12) thereon in such a way as to be opposed to each other and for moving the source (8) and the sensor (12) around the head of the patient (2);
the method comprising the use of a processing unit (16) for performing a shift-and-add-operation on the frames (14) for generating the digital panoramic image (20) of a focal plane (34) in the head region of the patient (2),
**characterized in that**
in the digital shift-and-add operation, the frames (14) are weighted by weighting coefficients obtained from a weighting function (42) having, along a line extending across the frame (14) in the moving direction (30) of the sensor (12), a maximum in a central region of the frame (14) and smaller values towards the edge of the frame (14).

2. Method according to claim 1,
wherein the weighting function (42) comprises a maximum along a central vertical line (47) of the frames (14) and decreases to smaller values towards vertical boundaries (48) of the frames (14).

3. Method according to claim 1 or 2,
wherein the weighting function (42) is a two-dimensional weighting function (42) that varies in the horizontal and vertical direction.

4. Method according to any one of claims 1 to 3,
wherein the slopes of the weighting function (42) decreasing from a central maximum (50) towards the vertical boundaries (48) along a horizontal line are symmetric or asymmetric with respect to the maximum.

5. Method according to any one of claims 1 to 4,
wherein the weighting functions (42) associated with frames (14) that are taken at positions, which are symmetric with respect to a median plane (36) of the patient (2), are identical or mirrored with respect of the median plane (36).

6. Method according to any one of claims 1 to 5,
wherein the weighting function (42) is exclusively applied to frames (14) taken in a region around the median plane (36) of the patient (2).

7. Method according to claim 6,
wherein the weighting function (42) is applied to frames (14), in which the angle between the normal of the sensor (12) in the associated position and the median plane (36) is in a range extending from 0° to an upper limit lying between 30° to 40°.

8. Method according to any one of claims 1 to 6,
wherein the full-width-half-maximum (FWHM) of the weighting function (42) is at its minimum for the frame (14) at the position where the scan trajectory of the sensor (12) intersects the median plane (36) of the patient (2).

9. Method according to claim 8,
wherein the FWHM of subsequent weighting functions (42) decreases gradually from one weighting function (42) to the next weighting function (42) towards the minimum, or wherein the FWHM decreases in a single step or in multiple steps to the minimum, wherein the FWHM remains constant between the steps.

10. Method according to any one of claims 1 to 9,
wherein frame columns (43) are weighted by multiplying pixel values of the frame columns (43) with a single weighting coefficient provided by the weighting function (42) for the respective frame column (43).

11. Method according to any one of claims 1 to 10,
wherein the shift-and-add operation comprises the operations:
- aligning the frame (14) with the panoramic image (20);
- weighting the pixel values of the frame (14); and
- adding the weighted pixel values to the panoramic pixels (31) of the panoramic image (20).

12. Method according to any one of claims 1 to 11,
wherein the frame (14) is aligned by shifting the frame (14) to a determined or predetermined position on the panoramic image (20), and
wherein the pixel values of the frame (14) are added to panoramic pixel values of corresponding panoramic pixels (31) of the panoramic image (20) or wherein, in case of a pixel mismatch between pixels of the frame (14) and panoramic pixels (31), by distributing the pixel values of a frame column (43) to the columns (45) of the panoramic image (20), which are overlapped by the frame column (43), wherein the splitting is performed by splitting the values of the frame column (43) proportional to the offset in the moving direction of the sensor (12), and
wherein the pixel values of the frame (14) are weighted before or after aligning the frame (14).

13. Method according to any one of claims 1 to 11,
wherein the frame (14) is aligned by interpolating the pixel values of the frame (14) along a frame line (44) and where the interpolation function (49) is used to resample the pixel values of the frame (14) to the panoramic pixels (31), and
wherein the original pixel values of the frame (14) before interpolation or the resampled pixel values of the frame (14) after interpolation are weighted.

14. Method according to any one of claims 1 to 13,
wherein the movement between two consecutive frames (14) is not less than half the pixel width and stays less than the full width of the pixel (13), wherein the pixel width is the dimension of the pixels (13) in the moving direction (30) of the sensor (12).

15. Method according to any one of claims 1 to 14,
wherein the patient's head region to be imaged is the dentition.

16. Apparatus for digital panoramic imaging in the head region of a patient (2) comprising:
- a source (8), which generates X-ray radiation;
- a sensor (12) provided with a two-dimensional array of pixels (13), which are suitable for generating frames (14) based on the X-ray radiation, which is generated by the source (8) and passes through the patient (2);
- a rotary unit (6) for arranging the source (8) and the sensor (12) thereon in such a way as to be opposed to each other;
- a supporting structure (3, 4, 5, 6) for supporting the rotary unit (6), wherein a motor driven translation unit (4) and a motor driven rotation unit (5) are interposed between the rotary unit (6) and the supporting structure (3) for moving the source (8) and the sensor (12) around the patient's head; and
- a processing unit (16) arranged for performing a shift-and-add-operation on the frames (14) for generating a digital panoramic image (20) of a focal plane (34) in the head region of the patient (2);
**characterized in that**
the processing unit (16) is arranged for performing a method according to any one of claims 1 to 15.

17. Apparatus according to claim 16,
wherein the supporting structure comprises:
- a rotary unit (6) for arranging the source (8) and the sensor (12) thereon in such a way as to be opposed to each other; and
- a support (3) for supporting the rotary unit (6), wherein a motor driven translation unit (4) and a motor driven rotation unit (5) are interposed between the rotary unit (6) and the supporting structure (3) for moving the source (8) and the sensor (12) around the head of the patient (2).

18. Apparatus according to claim 17,
wherein the apparatus comprises a control unit (15), which controls the source (8), the sensor (12), the translation unit (4) and the rotation unit (5) and which is provided with the processing means (16) arranged for performing a shift-and-add-operation on the frames (14).

19. Computer program product,
**characterized in that**
the computer program product contains program code for implementing any one of the methods according to any one of claims 1 to 15.
